# EUROPEAN PATENT SPECIFICATION

(11) **EP 3 873 347 B1**
(45) Date of publication and mention of the grant of the patent: **23.03.2022**
(21) Application number: 18849486.8
(22) Date of filing: 28.12.2018
(51) Int. Cl.: A61B 10/00, A61B 5/097

(54) **DEVICE FOR COLLECTING SAMPLES OF THE ALVEOLAR RESPIRATORY PORTION**
VORRICHTUNG ZUR ENTNAHME VON PROBEN DES ALVEOLÄREN RESPIRATIONSTEILS
DISPOSITIF DE COLLECTE D'ÉCHANTILLONS DE LA RÉGION RESPIRATOIRE ALVÉOLAIRE

(43) Date of publication of application: 08.09.2021
(73) Proprietor: Predict Srl, 70124 Bari (BA) (IT)
(72) Inventor: GIGANTE, Angelo Aurelio, 70056 Molfetta BA (IT); CARDANOBILE, Marco, 70124 Bari (BA) (IT); DE GENNARO, Gianluigi, 70056 Molfetta (BA) (IT)
(74) Representative: Braidotti, Andrea
(86) International application number: PCT/IT2018/000173
(87) International publication number: WO 2020/136685

(56) References cited:
- US-A- 5 465 728
- US-A1- 2005 177 056
- US-A1- 2015 335 267

## Description

The present invention relates to the breath sampling field, and in particular is a medical device for sampling the air contained in the last respiratory tract, called in the jargon: "End-Tidal".

US5465728 discloses a portable apparatus for collecting mammalian breath for chemical analysis and as a diagnostic tool for the physician. The apparatus comprises a fluid reservoir container having first and second ends and a body extending between these ends so as to define an interior chamber; a single breath entry portal; a breath exit portal; a sampling portal; a jacket to maintain the temperature of the chamber; a sample container for holding samples of exhaled breath; and pump means for moving selected samples of breath from the reservoir container into the sample container.

US2005177056 discloses an automatic breath collecting and sampling system, which is preferably carried by the subject, or otherwise kept with him or near him, for the recommended duration of the collection period. The system collects samples in separate switchable containers, either at predetermined time intervals, or in response to an automatic stimulus. After collection of the requisite number of separate containers, they may be detached and sent for analysis or other treatment.

US2015335267 discloses a complex apparatus for collecting volatile compounds in human breath and comprises a device for discriminating between alveolar and non-alveolar portions of exhaled breath by real-time measurement of a physical characteristic of said exhaled breath.

It is a main object of the device according to the invention, which will be referred to below as "sampler", to allow the doctor or the operator to collect two types of samples on two cartridges: a first cartridge Cp, on which the alveolar breath of a patient is collected, and a second cartridge Ca, containing the air of the environment in which the sampling is carried out.

According to the present invention, it is contemplated that once the acquisition step is completed, the two cartridges are sent to a specialized laboratory to be analyzed. The output of the analysis at the laboratory - appropriately anonymous - is automatically sent to the doctor who performed the examination and simultaneously uploaded to a remote database. The latter database collects data from all devices anonymously.

The process is structured as follows:
1. The patient goes to the sampling point (hospital, pharmacy, etc.), authorizing the medical staff to process his/her personal data;
2. The doctor prepares, within the device, the disposable components (nozzles and cartridges) which will be used for each patient and turns the device on;
3. The device automatically performs a purging procedure which allows cleaning of the device, ensuring greater reliability of the acquisition;
4. The doctor makes a medical history of the patient entering all the data concerning the patient's health status in the software;
5. The patient undergoes the examination by exhaling through a disposable mouthpiece present on the sampler;
6. The device collects the breath and ambient air samples on the cartridges Cp and Ca, respectively;
7. Once the analysis has been completed, the software allows the doctor to print the QR-Code identifying the sample, which is then applied to the cartridges ;
8. The two cartridges are sent to a specialized assay laboratory;
9. The laboratory analyzes the cartridges (without any reference to the patient's sensitive data), sends its report to the center where the sampling was carried out and uploads it to a remote centralized database;
10. The doctor displays the laboratory report and makes the diagnosis.

### OPERATING PRINCIPLE

In order to collect only the breath belonging to the desired alveolar tract on the Cp cartridge, there are adequate volume buffers to the patient, such as 200 ml for adults, lower for children. The patient is asked to make, through disposable mouthpieces, several deep expirations within the device: according to the invention, it is contemplated that the first part of the breath expelled by the patient passes through the device and exits therefrom; the last part of the breath, instead, remains collected within said buffers .

At this point, the solenoid valves inside the sampler are closed and a suction pump P is activated to cause the passage of air contained in the buffers through the first cartridge Cp and the consequent entrapment of the volatile organic compounds on the adsorbent bed contained in the cartridge itself. This mode of operation allows, asking the patient to exhale alternately in the two disposable mouthpieces, the two buffers B1 and B2 to be filled with breath: the device is configured so that the pump P, once activated, empties the buffer B1 first and then the buffer B2, pouring the contents into the cartridge Cp.

Advantageously, the sampler according to the present invention does not require medical air sources to be inhaled by the patient: the latter is required only to exhale inside the device.

The sections of the device in which the air exhaled by the patient flows are brought, through a specially designed heating system, to a temperature which prevents the formation of condensation which could compromise the assay (since the adsorbent materials of the cartridges are water-repellent, they prevent the formation of condensation inside the cartridges). For this purpose, a temperature sensor is also provided which is configured for measuring, at fixed time intervals, the temperature and for sending the measured value to a mini PC set up inside the sampler itself. Depending on the temperature detected, the mini PC automatically turns the heating system on or off.

As regards the second cartridge Ca, intended for ambient air, said solenoid valves are configurable (as will be seen hereafter) so as to allow, through the action of the pump P, the air intake of the surrounding environment in order to make it flow into the cartridge Ca.

The volume of breath and ambient air sent to the respective cartridges is instead determined by means of a flow meter F.

The device is managed by the user (doctor, technician, etc.) through its graphical interface, after proper two-factor authentication:
1. Software, through access credentials, to be entered on a graphical interface;
2. Hardware, via an authorized USB flash drive, to be connected to the sampler.

At the end of the acquisition, the system allows printing two copies of an identifying QR Code for the two cartridges Cp and Ca to be applied onto the same to send them to the assay center with an appropriate laboratory.

For each patient, the device provides a local encrypted database on which all useful (sensitive) data can be saved in order to identify the patient, his/her medical history and all the information related to the sampling performed. Once the two cartridges Cp and Ca have been received, identified by said anonymous QR codes with respect to the sensitive data of the sampled patients, the laboratory carries out the assay and, through an ad hoc software, sends his/her report to the remote database and simultaneously sends a notification (preferably by email) to the hospital center of origin of the cartridges. In this way, through the synchronization of the sampler via the internet, the doctor has the opportunity to view the report provided by the laboratory (and then proceed with his/her diagnostic procedure) and the remote database, at the same time, gradually populates an increasing amount of data.

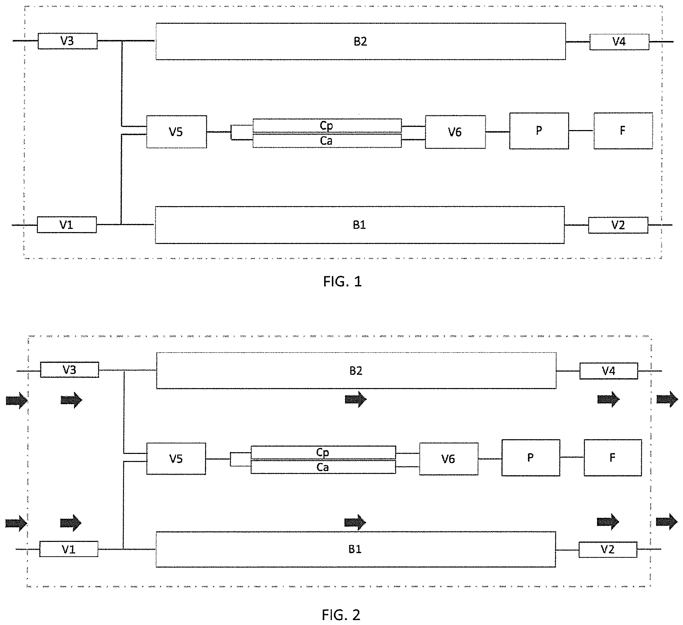

The sampling device 1 according to the invention provides a pneumatic circuit comprising (figure 1):
- VI, V2, V3, V4 = 2-way solenoid valves
- V5, V6 = 3-way solenoid valves
- Cp = patient cartridge
- Ca = ambient air cartridge
- P = suction pump
- F = flow meter
- BI, B2 = buffer

The volume of each buffer is preferably not greater than 200 ml, because this is the volume of alveolar tract which is expected to be sampled.

The main functions related to the operating steps are:

### I - Ambient air acquisition:

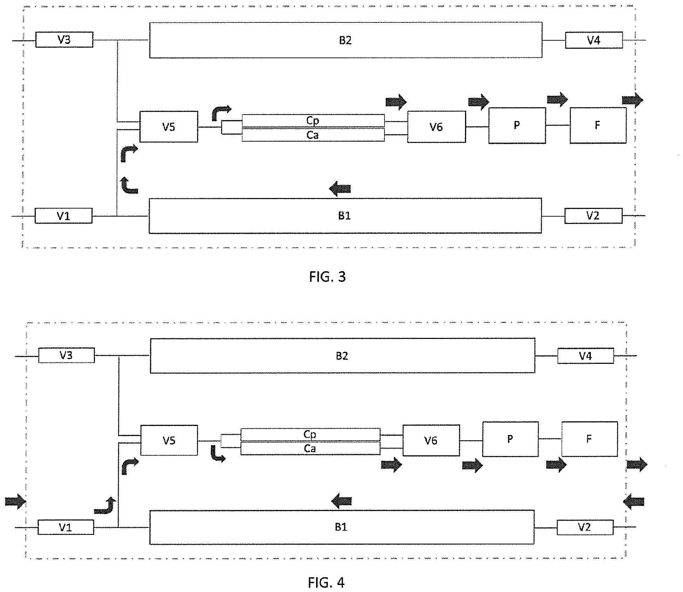

- The solenoid valves inside the device VI and V3 are closed and the solenoid valves V2 and V4 are open: the valve V5 is configurable to open one channel first and then another, so as to allow the pump P to suck in air from outside and send it to the cartridge Ca, making it pass first through buffer B1 and then through buffer B2
- The solenoid valve V6 is configured for connecting the pump P to the cartridge Ca.
- The suction pump P is activated and remains in the ON state until, through the feedback of the flow meter F, it is calculated that the required volume of air has been sent onto the cartridge Ca (Fig. 4)
- The ambient air enters the device through the solenoid valves V2 and V4 and passes through the buffers B1 and B2.

It is preferable to first acquire the ambient air in the cartridge Ca and then the patient's breath in the cartridge Cp: in this way, the ambient air is not contaminated by the patient's breath.

### II - Acquisition of the patient's breath:

- The patient exhales inside the sampling device 1 The bronchial breath (first respiratory tract) passes through the inlet valves VI-VB, passes through the buffers B1-B2 and exits the outlet valve (V2-V4) of the sampling device 1, while the alveolar portion is collected inside buffers BI- B2, which have a volume equal to the average value estimated for the alveolar section (Fig. 2)
- Through the suction pump P, the air contained in the buffers B1-B2 is conveyed onto the cartridge Cp. In fig. 3, the transfer of breath from buffer B1 is indicated, but the same procedure is also applied to buffer B2, in fact the patient is asked to exhale in the disposable mouthpiece connected to valve V3, filling the buffer B1 and then to exhale first into the disposable mouthpiece connected to valve VI, filling the buffer B2.
- The process is reiterated until the desired amount of breath is made to pass through the cartridge Cp (Fig. 3)
- The solenoid valve V5 may be configured to first open one channel and then another, so as to allow the pump P to first empty the buffer B1 and then the buffer B2, causing the breath to flow from one or the other buffer;
- The solenoid valve V6, instead, is placed between the pump P and the cartridges, and is configured - in this case - to put in communication the cartridge Cp and the pump itself.

It is preferable to provide two separate mouthpieces for different reasons:
- Decrease the execution time of the examination (in fact, in this way, by means of software, with single keys it is possible to double the breath brought into to cartridge: one for each cartridge)
- Have a backup line, if one stops working
- Being able to connect one buffer to the sensor line and the other to a cartridge
- In the case in which a single mouthpiece is provided, connected to the two buffers by a 3-way valve upstream of the two buffers, there would be some bends in the pipes which are not acceptable because each bend increases the impedance (resistance due to losses of the air flow load) detected by the patient when he/she exhales; by providing two independent lines, on the other hand, this phenomenon has been reduced and the effect of having two totally independent lines has also been obtained.

### III - Purging of the device:

- The authorized user replaces the two cartridges Ca and Cp with two tubes (supplied with the device) of the same size as the cartridges which allow the pneumatic circuit to be closed
- In this case, the solenoid valves V1-V2-V3-V4 are open and the valves V5 and V6 change continuously and alternately ON-OFF status (open-closed) in order to make air flow in all the conduits of the pneumatic circuit inside the device
- The suction pump P is activated and remains in the ON status for a certain time, in order to allow the passage of the ambient air in all the branches of the device and then carry out a purge of the device (the air flow is as indicated for the acquisition of ambient air)

According to a peculiar feature of the present invention, the two cartridges Ca and Cp present in the device allow detecting the differences between the ambient air, also breathed by the patient, and the exhaled of the patient himself, so as to be able to determine if a contamination is present in ambient air or is due to a patient's pathology.

The pneumatic circuit described thus far is managed/controlled by an electrical interface as shown in Figure 5, where:
- VI, V2, V3, V4, V5, V6 = solenoid valve
- P = suction pump
- F = flow meter
- H = heating element
- T = temperature sensor
- Power supply outside the device

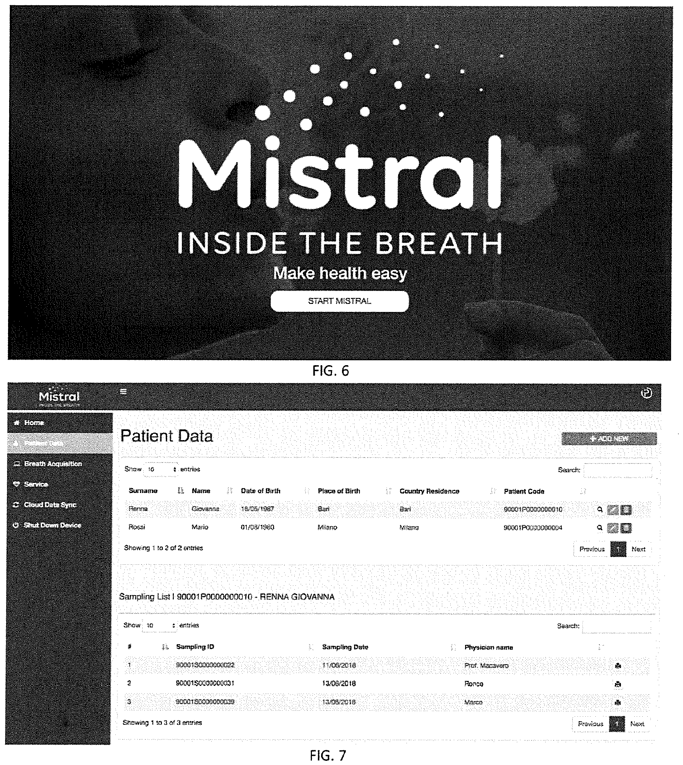

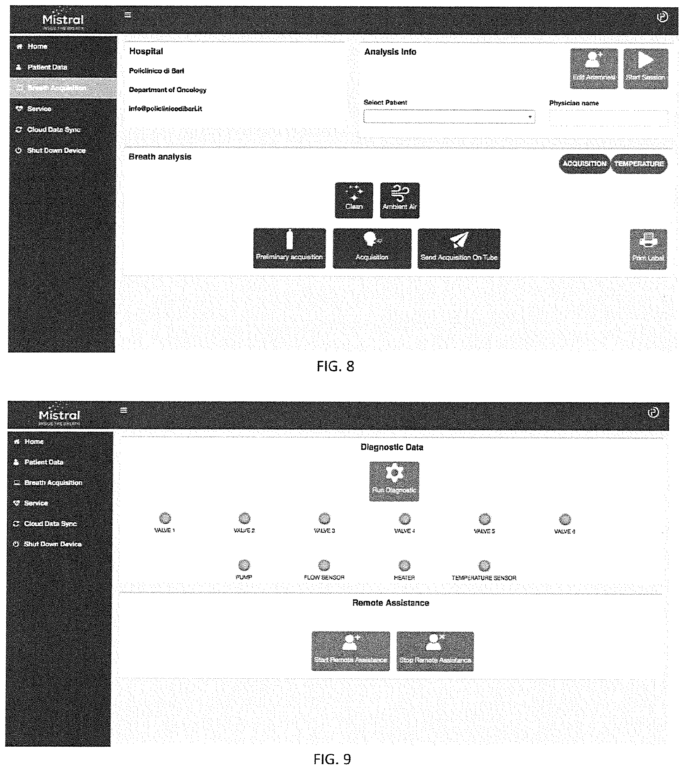

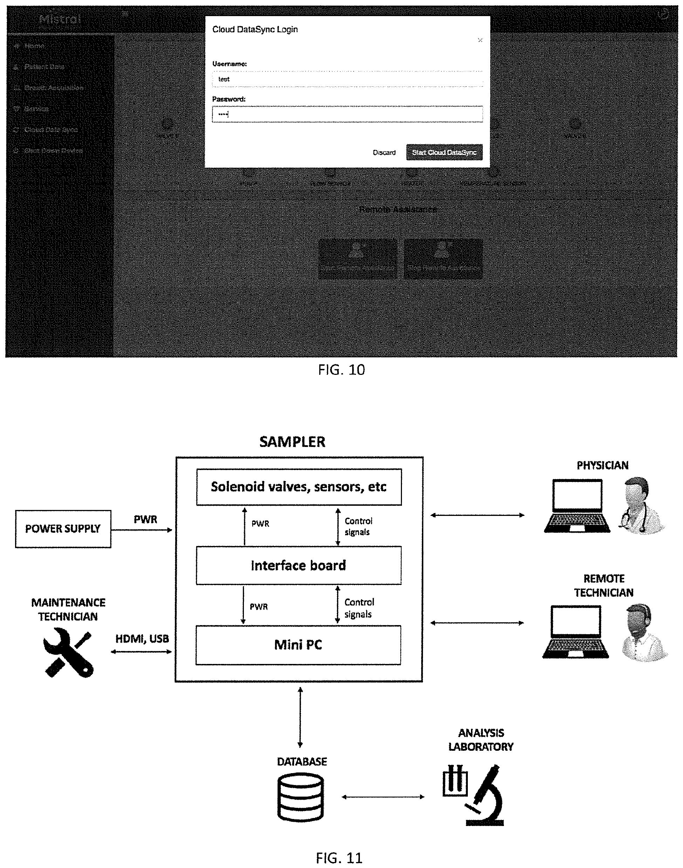

The graphical interface of the device user software - which can only be managed by authorized personnel - consists preferably of five main pages, shown as examples in figures 6 to 10:
- **Home page**: welcome page of the device (figure 6)
- **Patient data**: allows viewing, modifying or deleting one or more patients already present in the local database of the device, or adding new ones; By selecting a patient already entered, it is possible to view the list of samplings which have been already done (figure 7)
- **Breath Acquisition** (figure 8) allows:
   - selecting the patient to be sampled
   - entering data related to sampling
   - managing the various acquisition steps of the device
   - checking, by means of an LED, whether the temperature inside the device allows the examination to be carried out or not
   - checking, by means of an LED, whether the acquisition of the breath sample is finished or not o generating, at the end of the examination, a label (in PDF format) to be printed and applied on the cartridges: such a label contains a QR Code showing some details about the sampling done.
- **Service** (figure 9): the sampler autonomously and cyclically checks the correct functioning of all its components and indicates any anomalies to the user through green or red LEDs. The management/support of the device may also be carried out remotely due to the presence of two keys which allow to enable/disable the remote connection of a user provided with access credentials to the device.
- **Cloud Data Sync** (figure 10): after entering the authentication credentials, the device, if connected to an internet network, through a secure connection, synchronizes the local database with the remote one.

According to a peculiar feature of the present invention, the Cloud Data Sync is also configured to save data anonymously on a single remote DB; this feature allows carrying out - for example - epidemiological investigations.

Based on the foregoing, the device according to the present invention consists substantially of the following main components:
- **External body**: external part of the device, made of plastic material with the following provisions:
   - holes for the disposable mouthpiece connections o side handles to facilitate the patient's grip on the device
   - power button
   - connector for the power supply of the device o LED panel: contains LEDs indicating the operating status of the device
- **Sterile disposable mouthpieces**: made of plastic material and provided with check valves allow the patient to exhale inside the device;
- **Cp cartridge**: stainless steel cartridge containing an appropriate adsorbent bed on which the substances contained in the air exhaled by the patient are collected;
- **Cartridge Ca**: stainless steel cartridge containing an appropriate adsorbent bed on which the substances contained in the ambient air are collected;
- **Solenoid valves**: allow the flow of exhaled air to be guided inside the ducts, according to the operating step of the device;
- **Heating system**: useful to achieve, within the device, the optimal temperature to perform the examination;
- **Temperature sensors**: allow monitoring of the temperature inside the device and consequently controlling the heating system;
- **Flow sensor**: necessary to calculate the volume of breath collected on the cartridges;
- **Suction pump**: useful to suck the air either from the outside towards the cartridge Ca or from the buffers towards the cartridge Cp;
- **Power supply**;
   - **Mini PC**: it contains the management software of the device and generates the Wi-Fi Hotspot to which the personnel in charge of carrying out the examination is connected via external devices;
- **Interface card**: an electronic card which powers the whole system and allows connecting the Mini PC to the sensors and actuators;
- **Pneumatic fittings**: ducts through which the air exhaled by the patient and the ambient air flow;
- **Buffer of inert material and variable volume**: two single-use sachets in which the alveolar breath remains before it is brought onto the cartridge Cp. The size of these buffers varies according to the age of the patients: larger sachets for adults, smaller for children.

In figure 11 the system architecture is shown.

It should be noted that the process illustrated herein would be equally valid if the data collected by the sampler were transmitted to the assay laboratory by any other means (e.g. mail, paper mail, certified e-mail, etc.), ensuring in any case the anonymity of the patient's data.

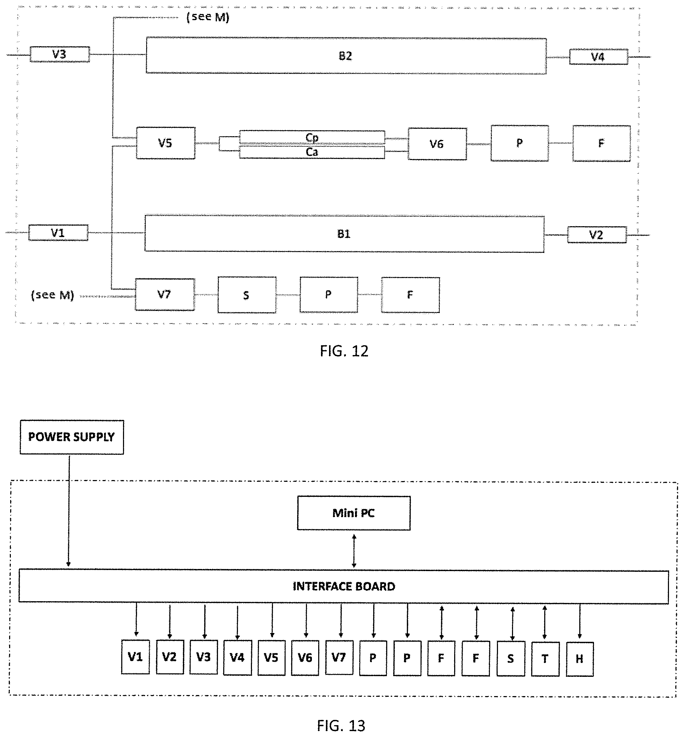

Within the device it is possible to integrate an additional acquisition line useful for analyzing in real time the sample of alveolar exhalation with an array of sensors. These sensors can, for example, be used either to verify the reproducibility of the acquisition of the exhaled sample or for diagnostic purposes, by measuring the amount of a given target molecule within the breath. The extended product architecture is therefore shown in Figure 12, where:
- VI, V2, V3, V4 = 2-way solenoid valves
- V5, V6, V7 = 3-way solenoid valves
- Cp = patient cartridge
- Ca = ambient air cartridge
- P = suction pump
- F = flow meter
- BI, B2 = buffer
- S = sensor array

The wiring diagram thus becomes that shown in figure 13, where:
- VI, V2 , V3 , V4, V5 , V6, V7 = solenoid valve
- P = suction pump
- F = flow meter
- H = heating element
- T = temperature sensor
- S = sensor array
- Power supply preferably, but not exclusively, outside the device.

## Claims

1. A sampling device (1) for collecting samples of the alveolar respiratory portion comprising
a pneumatic circuit managed/controlled by an electrical interface, wherein said pneumatic circuit comprises:
- A plurality of 2-way solenoid valves (V1, V2, V3, V4)
- At least one patient breath cartridge (Cp)
- At least one ambient air cartridge (Ca)
- At least one suction pump (P)
- At least one flow meter (F)
- At least a first buffer (B1)
wherein said cartridges (Ca and Cp) are configured to allow the identification of the differences between the ambient air, also breathed by the patient, and the breath of the patient him/herself, so as to be able to determine whether a contamination is present in the ambient air or it is due to a pathology of the patient;
wherein said electrical interface is operatively connected to said pneumatic circuit and further comprises:
- At least one heating element (H),
- At least one temperature sensor (T),
- At least one power supply outside or inside the device itself,
and **characterized in that** the device:
- comprises a plurality of 3-way solenoid valves (V5, V6),
- comprises at least a second buffer (B2)
- comprises two disposable mouthpieces so that the patient can exhale alternately in said two disposable mouthpieces to fill the two buffers B1 and B2 with breath,
- is configured so that the pump P, once activated, empties the buffer B1 first and the buffer B2, pouring the contents into the patient breath cartridge (Cp).

2. A sampling device (1) according to claim 1, **characterized in that** one of the three-way solenoid valve (V6) is fluidically connected to:
- said at least one patient breath cartridge (Cp)
- said at least one ambient air cartridge (Ca)
- said suction pump (P).

3. A sampling device (1) according to claim 2, **characterized in that** another three-way solenoid valve (V5) is fluidically connected to:
- said at least one patient breath cartridge (Cp) and said at least one ambient air cartridge (Ca) that are in parallel,
- said first buffer (B1), and
- said second buffer (B2).

4. A sampling device (1) according to one of the preceding claims, **characterized in that** it is provided with means for implementing a graphical interface of the software for its use, which consists of five main pages:
• Home page: welcome page of the device
• Patient data: configured to view, modify or delete one or more patients already present in the local database of the device, or to add new ones;
• Breath Acquisition: configured to allow:
∘ selecting the patient to be sampled
∘ entering data related to sampling o managing the various acquisition steps of the device
∘ checking, by means of an LED, whether the temperature inside the device allows the examination to be carried out or not
∘ checking, by means of an LED, whether the acquisition of the breath sample is finished or not
∘ generating, at the end of the examination, a label (in PDF format) to be printed and applied on the cartridges: such a label contains a QR Code showing some details about the sampling done.
• Service: configured so that the sampler can autonomously and cyclically perform a check on the correct functioning of all its components and can indicate to the user, through green or red LEDs, any anomalies; there are also two keys to enable/disable remote connection of a user with access credentials to the device, in order to provide remote management/support of the device;
• Cloud Data Sync: configured to synchronize a local database with a single remote DB, after entering the authentication credentials, through a secure connection which is connectable to an internet network; as well as to save data anonymously on said single and remote DB.

5. A sampling device (1) according to one of the preceding claims, **characterized in that** it substantially consists of the following main components:
• External body: external part of the device, made of plastic material with the following provisions:
∘ holes for disposable mouthpiece connections,
∘ side handles to facilitate the patient's grip on the device
∘ power button
∘ connector for the power supply of the device
∘ LED panel containing LEDs indicating the operating status of the device
• wherein the two disposable mouthpieces are sterile and made of plastic material and provided with check valves to allow the patient to exhale inside the device;
• wherein said patient breath cartridge (Cp) is made of stainless steel containing an appropriate adsorbent bed on which the substances contained in the air exhaled by the patient can be collected;
• wherein said ambient air cartridge (Ca) is made of stainless steel containing an appropriate adsorbent bed onto which the substances contained in the ambient air can be collected;
• wherein said solenoid valves (V1, V6) are configured to guide the flow of exhaled air into appropriate ducts, according to the operating step of the device;
• wherein said at least one heating element (H) is configured to achieve, within the device, the optimal temperature to perform the examination;
• wherein said at least one temperature sensor (T) is configured to allow monitoring of the temperature inside the device and control of the heating system;
• wherein said at least one flow meter (F) is configured to calculate the volume of breath/air collected on the cartridges;
• wherein said at least one suction pump (P) is configured to suck the air either from the outside towards the cartridge Ca or from the buffers towards the cartridge Cp;
• Power supply to supply electricity to the device;
• Mini PC: configured to contain and implement the device management software and to generate a Wi-Fi Hotspot to which external devices of the personnel assigned to carry out the examination can be connected ;
• Interface card: comprising an electronic card to power the whole system and to connect the Mini PC to the sensors and actuators;
• Pneumatic fittings: comprising ducts through which the air exhaled by the patient and the ambient air can flow;
• wherein said buffers (B1, B2) are made of inert material and of variable volume: two single-use sachets in which the alveolar breath remains before it is brought on the cartridge (Cp) ; the size of these buffers being variable according to the age of the patients: larger sachets for adults, smaller for children.

6. A sampling device (1) according to one of the preceding claims, **characterized in that** it provides an additional acquisition line for analyzing, in real time, a breath sample with a special array of sensors; wherein these sensors can be used to verify the reproducibility of the acquisition of the breath sample or for diagnostic purposes, by measuring the quantity of a given target molecule inside the breath, and wherein said acquisition line is configured to directly receive the alveolar portion of the patient's breath collected in at least one of the buffers (B1, B2) .

7. A sampling device (1) according to claim 5, **characterized in that** said temperature sensor is configured to measure, at fixed time intervals, the temperature and send the measured value to the mini PC, which in turn is configured to switch on or off automatically the heating system.

8. A method for purging the sampling device (1) according to one or more of the preceding claims, **characterized in that**:
- the two cartridges (Ca and Cp) are replaced with two tubes, supplied with the device, of the same size as the cartridges which allow the pneumatic circuit to be closed;
- the 2-way solenoid valves (V1-V2- V3-V4) are in open configuration and the 3-way solenoid valves (V5 and V6) are configured to continuously and alternately change an ON-OFF status (open-closed) in order to flow air in all the ducts of the pneumatic circuit within the device;
- The suction pump (P) is in active configuration and remains such for a predetermined time, in order to allow the passage of the ambient air in all branches of the device and then carry out a purge of the device.

## Patentansprüche

1. Probenentnahmevorrichtung (1) zum Sammeln von Proben des alveolären Respirationsteils, umfassend einen pneumatischen Kreislauf, der durch eine elektrische Schnittstelle verwaltet/gesteuert wird, wobei der pneumatische Kreislauf umfasst:
- eine Vielzahl von 2-Wege-Magnetventilen (V1, V2, V3, V4)
- mindestens eine Patientenatemkartusche (Cp)
- mindestens eine Umgebungsluftkartusche (Ca)
- mindestens eine Saugpumpe (P)
- mindestens einen Durchflussmesser (F)
- mindestens einen ersten Puffer (B1)
wobei die Kartuschen (Ca und Cp) dazu ausgelegt sind, die Identifikation der Unterschiede zwischen der Umgebungsluft, die auch vom Patienten geatmet wird, und dem Atem des Patienten selbst zu ermöglichen, um in der Lage zu sein, zu bestimmen, ob eine Kontamination in der Umgebungsluft vorliegt oder auf einer Pathologie des Patienten beruht;
wobei die elektrische Schnittstelle mit dem pneumatischen Kreislauf wirkverbunden ist und ferner umfasst:
- mindestens ein Heizelement (H),
- mindestens einen Temperatursensor (T),
- mindestens eine Stromversorgung außerhalb oder innerhalb der Vorrichtung selbst
und **dadurch gekennzeichnet, dass** die Vorrichtung:
- eine Vielzahl von 3-Wege-Magnetventilen (V5, V6) umfasst,
- mindestens einen zweiten Puffer (B2) umfasst
- zwei Einwegmundstücke umfasst, sodass der Patient abwechselnd in die zwei Einwegmundstücke ausatmen kann, um die zwei Puffer B1 und B2 mit Atem zu füllen,
- derart ausgelegt ist, dass die Pumpe P, sobald aktiviert, den Puffer B1 zuerst und den Puffer B2 leert und die Inhalte in die Patientenatemkartusche (Cp) füllt.

2. Probenentnahmevorrichtung (1) nach Anspruch 1, **dadurch gekennzeichnet, dass** eines der Drei-Wege-Magnetventilen (V6) fluidisch verbunden ist mit:
- der mindestens einen Patientenatemkartusche (Cp)
- der mindestens einen Umgebungsluftkartusche (Ca)
- der Saugpumpe (P).

3. Probenentnahmevorrichtung (1) nach Anspruch 2, **dadurch gekennzeichnet, dass** ein anderes Drei-Wege-Magnetventil (V5) fluidisch verbunden ist mit:
- der mindestens einen Patientenatemkartusche (Cp) und der mindestens einen Umgebungsluftkartusche (Ca), die parallel sind,
- dem ersten Puffer (B1) und
- dem zweiten Puffer (B2).

4. Probenentnahmevorrichtung (1) nach einem der vorstehenden Ansprüche, **dadurch gekennzeichnet, dass** sie mit einem Mittel zum Implementieren einer grafischen Schnittstelle der Software für ihre Verwendung versehen ist, die aus fünf Hauptseiten besteht:
- Startseite: Willkommensseite der Vorrichtung
- Patientendaten: dazu ausgelegt, einen oder mehrere Patienten, die bereits in der lokalen Datenbank der Vorrichtung vorhanden sind, anzuzeigen, zu modifizieren oder zu löschen oder neue hinzuzufügen;
- Atemaufnahme: dazu ausgelegt, zu ermöglichen:
• Auswählen des Patienten, von dem eine Probe entnommen werden soll
• Eingeben von Daten in Bezug auf die Probenentnahme oder Verwalten der verschiedenen Aufnahmeschritte der Vorrichtung
• Prüfen, mittels einer LED, ob es die Temperatur innerhalb der Vorrichtung ermöglicht, die Untersuchung auszuführen oder nicht
• Prüfen, mittels einer LED, ob die Aufnahme der Atemprobe abgeschlossen ist oder nicht
• Erzeugen, am Ende der Untersuchung, eines Etiketts (im PDF-Format), das ausgedruckt und auf den Kartuschen angebracht werden soll: ein solches Etikett enthält einen QR-Code, der einige Details über die erfolgte Probenentnahme zeigt.
- Dienst: derart ausgelegt, dass der Probenentnehmer autonom und zyklisch eine Prüfung der korrekten Funktionsweise aller seiner Komponenten durchführen kann und dem Benutzer, durch grüne oder rote LEDs, Anomalien angeben kann; es sind auch zwei Tasten zum Aktivieren/Deaktivieren einer Fernverbindung eines Benutzers mit Zugriffsanmeldeinformationen zu der Vorrichtung vorhanden, um eine Fernverwaltung/-unterstützung der Vorrichtung bereitzustellen;
- Cloud-Daten-Sync: dazu ausgelegt, eine lokale Datenbank mit einer einzigen Fern-DB nach dem Eingeben der Authentifizierungsanmeldeinformationen durch eine sichere Verbindung, die mit einem Internetnetzwerk verbindbar ist, zu synchronisieren sowie Daten anonym auf der einzigen und Fern-DB zu speichern.

5. Probenentnahmevorrichtung (1) nach einem der vorstehenden Ansprüche, **dadurch gekennzeichnet, dass** sie im Wesentlichen aus den folgenden Hauptkomponenten besteht:
- Außenkörper: Außenteil der Vorrichtung, hergestellt aus Kunststoffmaterial mit den folgenden Bereitstellungen:
• Löcher für Einwegmundstückanschlüsse,
• Seitengriffe zum Unterstützen des Greifens der Vorrichtung durch den Patienten
• Netzschalter
• Steckverbinder für die Stromversorgung der Vorrichtung
• LED-Feld, das LEDs enthält, die den Betriebsstatus der Vorrichtung angeben
- wobei die zwei Einwegmundstücke steril sind und aus Kunststoffmaterial hergestellt sind und mit Rückschlagventilen versehen sind, um es dem Patienten zu ermöglichen, innerhalb der Vorrichtung auszuatmen;
- wobei die Patientenatemkartusche (Cp) aus Edelstahl hergestellt ist und ein geeignetes Adsorptionsbett enthält, auf dem die Substanzen, die in der durch den Patienten ausgeatmeten Luft enthalten sind, gesammelt werden können;
- wobei die Umgebungsluftkartusche (Ca) aus Edelstahl hergestellt ist und ein geeignetes Adsorptionsbett enthält, auf dem die Substanzen, die in der Umgebungsluft enthalten sind, gesammelt werden können;
- wobei die Magnetventile (V1, V6) dazu ausgelegt sind, den Strom ausgeatmeter Luft gemäß dem Betriebsschritt der Vorrichtung in geeignete Kanäle zu führen;
- wobei das mindestens eine Heizelement (H) dazu ausgelegt ist, innerhalb der Vorrichtung die optimale Temperatur zur Durchführung der Untersuchung zu erreichen;
- wobei der mindestens eine Temperatursensor (T) dazu ausgelegt ist, ein Überwachen der Temperatur innerhalb der Vorrichtung und Steuern des Heizsystems zu ermöglichen;
- wobei der mindestens eine Durchflussmesser (F) dazu ausgelegt ist, das Volumen von auf den Kartuschen gesammeltem Atem/gesammelter Luft zu berechnen;
- wobei die mindestens eine Saugpumpe (P) dazu ausgelegt ist, die Luft entweder von außen in Richtung der Kartusche Ca oder vom Puffer in Richtung der Kartusche Cp zu saugen;
- Stromversorgung zur Versorgung der Vorrichtung mit Strom;
- Mini-PC: dazu ausgelegt, die Vorrichtungsverwaltungssoftware zu enthalten und zu implementieren und einen Wi-Fi-Hotspot zu erzeugen, mit dem externe Vorrichtungen des mit der Ausführung der Untersuchung beauftragten Personals verbunden werden können;
- Schnittstellenkarte: umfassend eine elektronische Karte zum Speisen des gesamten Systems und zum Verbinden des Mini-PC mit den Sensoren und Aktuatoren;
- Pneumatische Anschlüsse: umfassend Kanäle, durch die die vom Patienten ausgeatmete Luft und die Umgebungsluft strömen kann;
- wobei die Puffer (B1, B2) aus inertem Material hergestellt sind und von variablem Volumen sind: zwei Einwegbeutel, in denen der alveoläre Atem bleibt, bevor er auf die Kartusche (Cp) gebracht wird; wobei die Größe dieser Puffer gemäß dem Alter der Patienten variabel ist: größere Beutel für Erwachsene, kleinere für Kinder.

6. Probenentnahmevorrichtung (1) nach einem der vorstehenden Ansprüche, **dadurch gekennzeichnet, dass** sie eine zusätzliche Aufnahmeleitung zum Analysieren, in Echtzeit, einer Atemprobe mit einem speziellen Array von Sensoren bereitstellt; wobei diese Sensoren zum Verifizieren der Wiederholbarkeit der Aufnahme der Atemprobe oder für diagnostische Zwecke durch Messen der Menge eines gegebenen Zielmoleküls innerhalb des Atems verwendet werden können und wobei die Aufnahmeleitung dazu ausgelegt ist, den alveolären Teil des Atems des Patienten, der in mindestens einem der Puffer (B1, B2) gesammelt ist, direkt zu empfangen.

7. Probenentnahmevorrichtung (1) nach Anspruch 5, **dadurch gekennzeichnet, dass** der Temperatursensor dazu ausgelegt ist, die Temperatur in festen Zeitintervallen zu messen und den gemessenen Wert an den Mini-PC zu senden, der wiederum dazu ausgelegt ist, das Heizsystem automatisch ein- oder auszuschalten.

8. Verfahren zum Spülen der Probenentnahmevorrichtung (1) nach einem oder mehreren der vorstehenden Ansprüche, **dadurch gekennzeichnet, dass**:
- die zwei Kartuschen (Ca und Cp) durch zwei mit der Vorrichtung gelieferte Schläuche derselben Größe wie die Kartuschen ersetzt sind, die ein Schließen des pneumatischen Kreislaufs ermöglichen;
- die 2-Wege-Magnetventile (V1-V2- V3-V4) in einer offenen Konfiguration sind und die 3-Wege-Magnetventile (V5 und V6) dazu ausgelegt sind, fortlaufend und abwechselnd einen EIN-AUS-Status (offen-geschlossen) zu ändern, um Luft in alle Kanäle des pneumatischen Kreislaufs innerhalb der Vorrichtung strömen zu lassen;
- die Saugpumpe (P) in einer aktiven Konfiguration ist und für eine vorbestimmte Zeit so bleibt, um den Durchgang der Umgebungsluft in alle Zweige der Vorrichtung zu ermöglichen und dann eine Spülung der Vorrichtung auszuführen.

## Revendications

1. Dispositif de prélèvement (1) pour le prélèvement d'échantillons de la partie respiratoire alvéolaire comprenant un circuit pneumatique géré/contrôlé par une interface électrique, dans lequel ledit circuit pneumatique comprend :
- Une pluralité d'électrovannes à 2 voies (V1, V2, V3, V4)
- Au moins une cartouche respiratoire patient (Cp)
- Au moins une cartouche d'air ambiant (Ca)
- Au moins une pompe aspirante (P)
- Au moins un débitmètre (F)
- Au moins un premier tampon (B1)
dans lequel lesdites cartouches (Ca et Cp) sont configurées pour permettre l'identification des différences entre l'air ambiant, également respiré par le patient, et la respiration du patient lui-même, afin de pouvoir déterminer si une contamination est présente dans l'air ambiant ou elle est due à une pathologie du patient ;
dans lequel ladite interface électrique est fonctionnellement connectée audit circuit pneumatique et comprend en outre :
- Au moins un élément chauffant (H),
- Au moins une sonde de température (T),
- Au moins une alimentation externe ou interne à l'appareil lui-même,
et **caractérisé en ce que** le dispositif :
- comprend une pluralité d'électrovannes à 3 voies (V5, V6),
- comprend au moins un deuxième tampon (B2)
- comprend deux embouts jetables pour que le patient puisse expirer alternativement dans lesdits deux embouts jetables pour remplir en haleine les deux tampons B1 et B2,
- est configuré pour que la pompe P, une fois activée, vide d'abord le tampon B1 et le tampon B2, en versant le contenu dans la cartouche respiratoire du patient (Cp).

2. Dispositif de prélèvement (1) selon la revendication 1, **caractérisé en ce que** l'une des électrovannes à trois voies (V6) est reliée fluidiquement à :
- ladite au moins une cartouche d'haleine patient (Cp)
- ladite au moins une cartouche d'air ambiant (Ca)
- ladite pompe d'aspiration (P).

3. Dispositif de prélèvement (1) selon la revendication 2, **caractérisé en ce qu'**une autre électrovanne à trois voies (V5) est reliée fluidiquement à :
- ladite au moins une cartouche d'haleine patient (Cp) et ladite au moins une cartouche d'air ambiant (Ca) qui sont en parallèle,
- ledit premier tampon (B1), et
- ledit deuxième tampon (B2).

4. Dispositif de prélèvement (1) selon l'une des revendications précédentes, **caractérisé en ce qu'**il est pourvu de moyens de mise en oeuvre d'une interface graphique du logiciel pour son utilisation, qui se compose de cinq pages principales :
- Page d'accueil : page d'accueil de l'appareil
- Données patient : configuré pour visualiser, modifier ou supprimer un ou plusieurs patients déjà présents dans la base de données locale de l'appareil, ou pour en ajouter de nouveaux ;
- Acquisition du souffle : configuré pour permettre de :
• sélectionner le patient à prélever
• saisir les données liées à l'échantillonnage o gérer les différentes étapes d'acquisition de l'appareil
• vérifier, au moyen d'une DEL, si la température à l'intérieur de l'appareil permet ou non d'effectuer l'examen
• vérifier, au moyen d'une DEL, si l'acquisition de l'échantillon de souffle est terminée ou non
• générer, à la fin de l'examen, une étiquette (au format PDF) à imprimer et à appliquer sur les cartouches : une telle étiquette contient un QR Code donnant quelques détails sur le prélèvement effectué.
- Service : configuré pour que l'échantillonneur puisse effectuer de manière autonome et cyclique un contrôle du bon fonctionnement de tous ses composants et puisse signaler à l'utilisateur, par des DEL vertes ou rouges, toute anomalie ; il existe également deux clés pour activer/désactiver la connexion à distance d'un utilisateur avec des informations d'identification d'accès à l'appareil, afin de fournir une gestion/assistance à distance de l'appareil ;
- Cloud Data Sync : configuré pour synchroniser une base de données locale avec une seule base de données remote, après saisie des identifiants d'authentification, via une connexion sécurisée pouvant être connectée à un réseau Internet ; ainsi que de sauvegarder des données de façon anonyme sur ladite BD unique et distante.

5. Dispositif de prélèvement (1) selon l'une des revendications précédentes, **caractérisé en ce qu'**il est constitué sensiblement des composants principaux suivants :
- Corps externe : partie externe de l'appareil, réalisée en matière plastique avec les dispositions suivantes :
• trous pour raccords d'embouts jetables,
• poignées latérales pour faciliter la préhension du patient sur l'appareil,
• bouton d'alimentation,
• connecteur pour l'alimentation de l'appareil,
• Panneau DEL contenant des DEL indiquant l'état de fonctionnement de l'appareil,
- Dans lequel les deux embouts buccaux jetables sont stériles et en matière plastique et munis de clapets anti-retour pour permettre au patient d'expirer à l'intérieur de l'appareil ;
- dans lequel ladite cartouche d'haleine patient (Cp) est en acier inoxydable contenant un lit adsorbant approprié sur lequel les substances contenues dans l'air expiré par le patient peuvent être recueillies ;
- dans lequel ladite cartouche d'air ambiant (Ca) est en acier inoxydable contenant un lit d'adsorbant approprié sur lequel les substances contenues dans l'air ambiant peuvent être collectées ;
- dans lequel lesdites électrovannes (V1, V6) sont configurées pour guider le flux d'air expiré dans des conduits appropriés, selon l'étape de fonctionnement du dispositif ;
- dans lequel ledit au moins un élément chauffant (H) est configuré pour obtenir, à l'intérieur du dispositif la température optimale pour effectuer l'examen ;
- dans lequel ledit au moins un capteur de température (T) est configuré pour permettre la surveillance de la température à l'intérieur du dispositif et le contrôle du système de chauffage ;
- dans lequel ledit au moins un débitmètre (F) est configuré pour calculer le volume de respiration/air collecté sur les cartouches ;
- dans lequel ladite au moins une pompe d'aspiration (P) est configurée pour aspirer l'air soit de l'extérieur vers la cartouche (Ca) soit des tampons vers la cartouche (Cp) ;
- Alimentation électrique pour alimenter l'appareil en électricité ;
- Mini PC : configuré pour contenir et mettre en œuvre le logiciel de gestion des appareils et pour générer un Hotspot Wi-Fi auquel les appareils externes du personnel affecté à la réalisation de l'examen peuvent être connectés ;
- Carte d'interface : comprenant une carte électronique pour alimenter l'ensemble du système et connecter le Mini PC aux capteurs et actionneurs ;
- Raccords pneumatiques : constitués de conduits par lesquels peuvent circuler l'air expiré par le patient et l'air ambiant ;
- dans lequel lesdits tampons (BI, B2) sont en matériau inerte et de volume variable : deux sachets à usage unique dans lesquels séjourne le souffle alvéolaire avant qu'il ne soit amené sur la cartouche (Cp) ; la taille de ces tampons étant variable selon l'âge des patients : sachets plus gros pour les adultes, plus petits pour les enfants.

6. Dispositif de prélèvement (1) selon l'une des revendications précédentes, **caractérisé en ce qu'**il prévoit une ligne d'acquisition supplémentaire pour analyser, en temps réel, un échantillon d'haleine avec un réseau spécial de capteurs ; dans lequel ces capteurs peuvent être utilisés pour vérifier la reproductibilité de l'acquisition de l'échantillon d'haleine ou à des fins de diagnostic, en mesurant la quantité d'une molécule cible donnée à l'intérieur de l'haleine, et dans lequel ladite ligne d'acquisition est configurée pour recevoir directement la partie alvéolaire du haleine du patient recueillie dans au moins un des tampons (B1, B2) .

7. Dispositif de prélèvement (1) selon la revendication 5, **caractérisé en ce que** ledit capteur de température est configuré pour mesurer, à intervalles de temps fixes, la température et envoyer la valeur mesurée au mini PC, qui à son tour est configuré pour s'allumer ou éteint automatiquement le système de chauffage.

8. Procédé de purge du dispositif d'échantillonnage (1) selon une ou plusieurs des revendications précédentes, **caractérisé en ce que** :
- les deux cartouches (Ca et Cp) sont remplacées par deux tubes, fournis avec l'appareil, de même dimension que les cartouches qui permettent de fermer le circuit pneumatique ;
- les électrovannes 2 voies (V1-V2-V3-V4) sont en configuration ouverte et les électrovannes 3 voies (V5 et V6) sont configurées pour changer en continu et alternativement un état ON-OFF (ouvert-fermé) en faire circuler l'air dans toutes les gaines du circuit pneumatique à l'intérieur de l'appareil ;
- la pompe d'aspiration (P) est en configuration active et le reste pendant un temps prédéterminé, afin de permettre le passage de l'air ambiant dans toutes les branches de l'appareil puis d'effectuer une purge de l'appareil.
